# EUROPÄISCHE PATENTSCHRIFT

(11) **EP 1 847 292 B1**
(45) Veröffentlichungstag und Bekanntmachung des Hinweises auf die Patenterteilung: **02.09.2009**
(21) Anmeldenummer: 07006125.4
(22) Anmeldetag: 24.03.2007
(51) Int. Cl.: A61N 1/375, A61N 1/372

(54) **Aktives medizinisches implantierbares Gerät mit mindestens zwei diagnostischen und/oder therapeutischen Funktionen**
Active medical implant device with at least two diagnostic and/or therapeutic functions
Dispositif médical implantable actif doté d'au moins deux fonctions diagnostiques et/ou thérapeutiques

(30) Priorität: 22.04.2006 DE 102006018851
(43) Veröffentlichungstag der Anmeldung: 24.10.2007
(73) Patentinhaber: Biotronik CRM Patent AG, 6341 Baar (CH)
(72) Erfinder: Flach, Erhard, 12305 Berlin (DE); Geistert, Wolfgang, Dr., 79618 Rheinfelden (DE); Jacobsen, Roland, 12359 Berlin (DE); Schaldach, Max, Dr., 12359 Berlin (DE); Ulbrich, Axel, 12355 Berlin (DE)
(74) Vertreter: Lindner-Vogt, Karin L.

(56) Entgegenhaltungen:
- US-A- 5 411 535
- US-A- 5 810 735
- US-A- 5 919 213
- US-A- 6 141 588
- US-A1- 2005 055 056

## Beschreibung

Die Erfindung betrifft ein aktives medizinisches Geräteimplantat mit mindestens zwei diagnostischen und/oder therapeutischen Funktionen.

Aus dem Stand der Technik ist eine Vielzahl verschiedenartiger aktiver Implantate bekannt, wie beispielsweise Herzschrittmacher, Defibrillatoren, Insulinpumpen, Neurostimulatoren, Körperfunktionsaufzeichnungsgeräte (so genannte "Eventrecorder"), Herzunterstützungssysteme usw. Diesen Implantaten ist gemeinsam, dass sie für einen bestimmten Anwendungszweck ausgelegt sind und eine vom Gerätehersteller vorgegebene Funktionalität aufweisen. Letztere ist in der Regel auf eine bestimmte Erkrankung des Implantatträgers ausgerichtet, die durch das Implantat diagnostiziert und/oder therapiert werden soll.

Viele Patienten haben jedoch nicht nur eine bestimmte Erkrankung, sondern mehrere oder systemische Krankheitsbilder, die einer Diagnose oder Therapie bedürfen. Ferner besteht oftmals auch nur der Bedarf nach einer systemischen Überwachung. Dies ist mit einem vorgegebenen Implantat in der Regel schwierig umzusetzen, da die hierfür notwendigen Einzelkomponenten in einem systemischen Zusammenhang stehen und sich gegenseitig beeinflussen.

Jedoch ist beispielsweise aus der US 6,141,588 ein aktives medizinisches Geräteimplantat mit einem Basisgeräteteil in Form eines aktiven Implantats bekannt, das mit einer zentralen Steuereinheit und einem Bussystem ausgestattet ist. Gesonderte aktive Implantatmodule können über dieses Bussystem miteinander gekoppelt werden und mit der zentralen Steuereinheit kommunizieren und/oder durch diese gesteuert werden. Die Implantatmodule nehmen jeweils einen Messwert auf, eine auf diesem jeweiligen Modul befindliche Diagnoseeinheit wertet das Signal aus und sendet die Nachricht in Form eines verschlüsselten Signals über das Bussystem an das Basisgeräteteil, wo es dann von der zentralen Steuereinheit ausgewertet wird. Die zentrale Steuereinheit ermittelt daraufhin eine geeignete Therapie, welche dann wieder in Form eines Signals an das jeweilige Implantatmodul geleitet wird. Dort wird dann die Therapie ausgeführt.

Nachteilig an dieser Lösung ist, dass jedes einzelne Implantatmodul sozusagen ein "Spezialist" ist. Die Messwerte können nur von einer dem messenden Implantatmodul zugeordneten und auf diesem befindliche Diagnoseeinheit prozessiert werden, folglich also auch nur eine Untersuchung auf ein bestimmtes Krankheitsbild vornehmen. Erfolgt beispielsweise ein Messevent, der bezüglich des von der zugeordneten Diagnoseeinheit zu bestimmenden Krankheitsbildes völlig unverdächtig ist, der aber in Bezug auf andere Krankheitsbilder höchst kritisch erscheint, kann dieser eventuell kritische Zustand des Patienten nicht erkannt werden. Somit ist der hochflexible individuelle Einsatz eines solchen Implantats nicht möglich, da nur vordefinierte Zustände des Patienten ermittelt werden können. Weiterhin ist eine weitgehend sichere systemische Überwachung nicht gewährleistet, nämlich dann, wenn aufgrund eines Defektes die Diagnosefunktionalität der zentralen Steuereinheit ausfällt.

Aufgrund der geschilderten Problematik besteht also ein Bedürfnis für ein Implantat, das individuell auf ein komplexes Krankheitsbild oder Diagnosebedürfnis eines Patienten abstimmbar und so konfigurierbar ist, dass es im Bedarfsfall verschiedenartige Informationen ggf. an mehreren Stellen des Körpers aufnehmen, verarbeiten und wiederum ggf. darauf aufbauend eine angepasste Therapie ableiten und applizieren kann.

Aufgabe der vorliegenden Erfindung ist es deshalb, ein aktives medizinisches Geräteimplantat zu schaffen, das vom Arzt während der Implantationsprozedur und im Betrieb den individuellen Bedürfnissen eines Patienten entsprechend konfigurierbar ist und eine sichere systemische Überwachung bietet, ohne dabei technisch aufwändig zu sein.

Diese Aufgabe wird laut Patentanspruch 1 dadurch gelöst, dass die aktiven Implantatmodule des Geräteimplantats über das Bussystem zusätzlich untereinander uni-, bi- oder multidirektional kommunizieren.

Damit es nicht zu Messungenauigkeiten oder gar Messfehlern kommen kann, implementiert die zentrale Steuereinheit bei der Steuerung eine Master-and-Slave-Steuerung bezüglich der hierarchischen Ordnung der Implantatmodule.

Das bedeutet, dass die Implantatmodule noch unabhängiger von starren bekannten Implantaten werden, weshalb das Implantat auch noch leichter auf ein komplexes Krankheitsbild oder Diagnosebedürfnis eines Patienten einstellbar ist. Dann können auch wesentlich leichter und effektiver Informationen an mehreren Stellen beispielsweise verarbeitet bzw. diagnostiziert werden. Beispielsweise kann an einem Implantatmodul ein Messwert aufgenommen werden, der aber nicht im selben Modul verarbeitet werden kann, weil die Diagnosefunktion nicht in diesem, aber in einem anderen Modul vorhanden ist. Es ist weiter von Vorteil, dass der Messwert zwar in der Diagnoseeinheit des messenden Implantatmoduls ausgewertet wird, dann aber an eine Diagnoseeinheit eines anderen Implantatmoduls zur weiteren Verarbeitung weitergeleitet werden kann. Als Folge kann auch das Basisgeräteteil sehr klein gehalten werden, da zusätzliche Auswerteeinheiten dezentral vorhanden sind und nicht mehr im Basisgeräteteil verfügbar sein müssen.

Aufgrund des Modulsystems können die einzelnen Module jeweils für einen bestimmten Anwendungszweck ausgelegt sein, wie z. B. für die Messung physikalischer oder chemischer Parameter und darauf basierend für die Berechnung physiologischer Parameter zu Diagnosezwecken. Darauf aufbauend wiederum kann eine bestimmte Therapie von dem aktiven Geräteimplantat appliziert werden. Als Beispiel für solche physikalischen und chemischen Parameter können genannt werden:
- Körpertemperatur
- elektrische Werte (Spannung, Strom, Impedanz)
- Druck
- Beschleunigung
- optische Werte (Farbe, Lichtdurchlässigkeit)
- Viskosität
- pH-Wert
- Zeit

Daraus zu ermittelnde physiologische Parameter sind dann beispielsweise:
- Herzfrequenz
- Atemfrequenz
- Nerven- bzw. Hirnaktivität
- Sauerstoffsättigung
- Milchsäure-Konzentration
- Blutzucker-Gehalt
- Cholesterin-Gehalt
- Histamin-Gehalt
- Bewegung (Aktivität)

Daraus ableitbare Therapien sind beispielsweise:
- elektrische Stimulation (nieder- und hochenergetisch), wie z. B. Herzschrittmacher oder Defibrillator
- mechanische Stimulation
- Medikamentengabe (z. B. Insulinpumpe)

Grundsätzlich ist es auf der Basis dieses Konzeptes möglich, in einem Modul eine oder mehrere der vorstehend aufgeführten Funktionalitäten zu implementieren sowie Module mit Spezialaufgaben, wie beispielsweise die Stromversorgung der anderen Module oder die Kommunikation unter diesen vorzusehen. In diesem Zusammenhang kann es vorteilhaft sein, ein Basisgeräteteil vorzusehen, über das die Implantatmodule koppelbar sind. Damit ist das Gesamtsystem dann kompakt und "aufgeräumt" umsetzbar.

Das Basisgeräteteil nimmt funktionsübergreifende Grundelemente für die Geräteimplantate auf, wie eine zentrale Steuereinheit, eine Kommunikationseinheit und/oder eine Energieversorgungseinheit für die Implantatmodule.

Gemäß einer vorteilhaften Ausführungsform ist für die Kopplung der Module und insbesondere deren Steuerung und/oder Kommunikation ein Bussystem vorgesehen, das beispielsweise aus mehreren elektrischen Leitern aufgebaut oder auf der Basis der Lichtleitertechnik realisiert ist.

Das Bussystem kann dabei so ausgelegt sein, dass die Implantatmodule zu ihrer Kopplung signaltechnisch und mechanisch mit dem Bussystem verbindbar, vorzugsweise darauf aneinanderreihbar sind. Die Module werden also an das Bussystem "angedockt", wozu beispielsweise eine Hindurchführung des Bussystems durch die Einzelmodule unter Aneinanderreihung von Implantatmodulen vorzugsweise über Steckkopplungen vorgesehen ist.

Die Kopplung der Module kann jedoch auch über ein drahtloses Bussystem erfolgen, was besonders dann vorteilhaft ist, wenn die einzelnen Implantatmodule nach dem Koppeln keine kompakte, mechanische Einheit bilden sollen, sondern im Körper verteilt platziert werden sollen. Eine solche drahtlose Kommunikation kann beispielsweise mit einer Funktechnologie, wie "Blue Tooth", einer induktiven Kopplung oder einer galvanischen Kopplung über eine leitfähige Körperflüssigkeit realisiert werden.

Gemäß einer weiteren bevorzugten Ausführungsform kann ein Implantatmodul mit austauschbaren Teilmodulen bestückt werden. Letztere weichen vorzugsweise von der eigentlichen Funktionalität des Implantatmoduls ab, sodass letzteres in seinem Funktionsumfang variabel erweitert werden kann. Diese Ergänzung oder auch Umbestückung kann auch zu einem späteren Zeitpunkt nach dem ersten Einsetzen des Geräteimplantats erfolgen, ohne das komplette Implantat tauschen zu müssen.

Zusammenfassend erlaubt das erfindungsgemäße Geräteimplantat eine sehr flexible Anpassung an diagnostische und therapeutische Anforderungen, was auch nach einem Ersteinsetzen des Implantates noch mit begrenztem Aufwand und ohne das gesamte Implantat tauschen zu müssen, durchführbar ist.

Weitere Merkmale, Vorteile und Einzelheiten der Erfindung ergeben sich aus der nachfolgenden Beschreibung eines Ausführungsbeispieles anhand der beigefügten Zeichnungen. Es zeigen:
- Fig. 1: eine schematische Darstellung eines aktiven medizinischen Geräteimplantats in Modulbauweise,
- Fig. 2: eine perspektivische Darstellung eines solchen Geräteimplantats mit verschiedenen Implantatmodulen, sowie
- Fig. 3: eine ausschnittsweise Perspektivdarstellung eines Implantatmoduls.

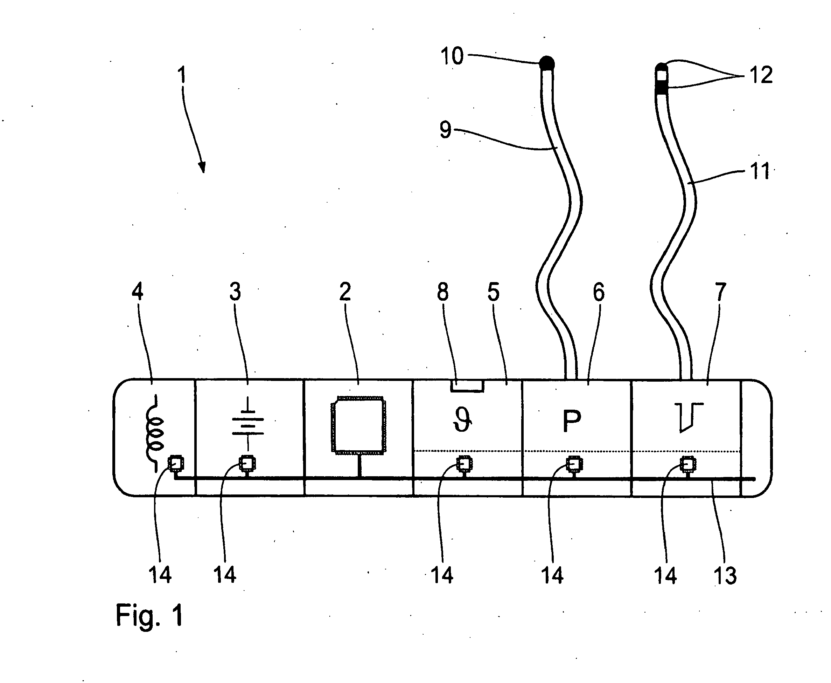

Anhand von Fig. 1 kann das grundsätzliche Konzept des medizinischen Geräteimplantats 1 erläutert werden. Dieses weist verschiedene Module auf, nämlich ein zentrales Steuermodul 2, ein Stromversorgungsmodul 3 und ein Kommunikationsmodul 4, das beispielsweise auf der Basis eines Transponders aufgebaut ist und über das das Geräteimplantat 1 mit einem externen Basisgerät kommunizieren kann. Ferner weist das Geräteimplantat 1 zwei diagnostische Messmodule 5, 6 zur Messung von Temperatur und Druck sowie ein therapeutisches Modul 7 zur elektrischen Stimulation auf. In das Messmodul 5, das der Temperaturmessung dient, ist ein Temperatursensor 8 integriert. Dieser nimmt bei einer Einpflanzung des Geräteimplantats in einen Patientenkörper die Körpertemperatur auf.

Das Messmodul 6 dient der Ermittlung des Drucks P in einem Körperteil und weist hierzu eine vom Messmodul 6 ausgehende Messleitung 9 auf, an deren freiem Ende ein Drucksensor 10 angebracht ist.

Das therapeutische Modul 7 weist einen angeschlossenen Katheter 11 auf, an dessen freiem Ende Stimulationselektroden 12 sitzen. Damit kann der Patientenkörper elektrisch stimuliert, also beispielsweise eine Herzschrittmacherfunktion realisiert werden.

Die Module 2 bis 7 sind gemeinsam über ein Bussystem 13 miteinander verbunden, das über Schnittstellen 14 an das jeweilige Modul 3 bis 7 signal- und energieversorgungstechnisch gekoppelt ist.

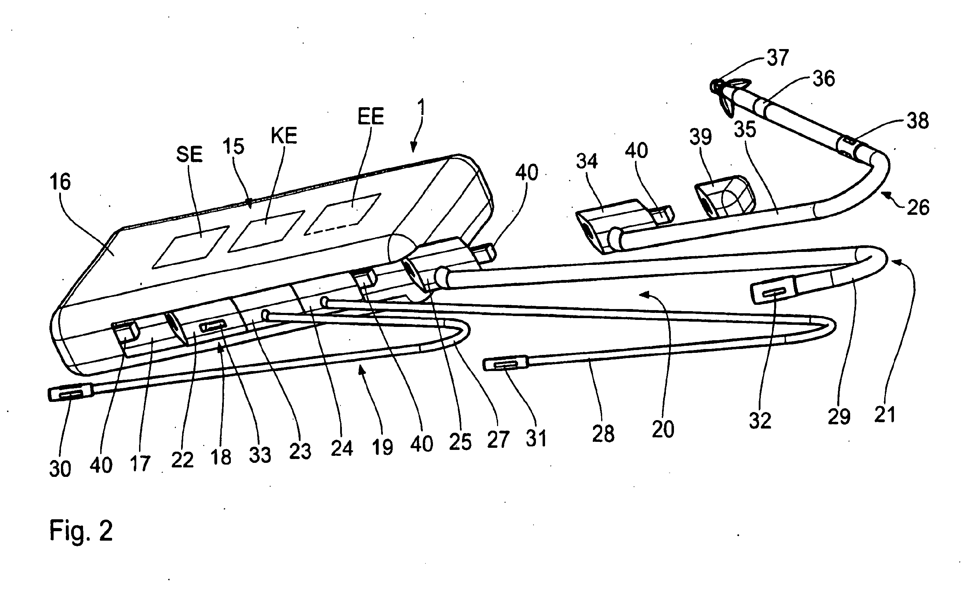

Anhand von Fig. 2 ist eine Umsetzung des in Fig. 1 schematisch dargestellten Modulsystems zu erläutern. So stellt ein Basisgeräteteil 15 das "Rückgrat" des medizinischen Geräteimplantats 1 dar. In diesem Basisgeräteteil 15 sind eine zentrale Steuereinheit SE, eine Kommunikationseinheit KE sowie eine Energieversorgungseinheit EE als funktionsübergreifende Grundelemente fest eingebaut. In dem gezeigten Ausführungsbeispiel sind nun diagnostische und therapeutische Module zur Ankopplung an das Basisgeräteteil 15 vorgesehen. Dazu weist das Basisgeräteteil 15 in der langen Schmalseite seines Gehäuses 16 eine Kopplungsschiene 17 auf, die durch eine entsprechende Gehäusenut gebildet ist. In diese sind mechanisch die verschiedenen dargestellten Module, nämlich beispielsweise die Messmodule 18, 19, 20, 21 mit ihrem jeweiligen Modulkopf 22 bis 25 einschiebbar. Die Modulköpfe 22 bis 24 sind dabei in Fig. 2 in einer Stellung gezeichnet, in der sie noch nicht bis zum Ende der Kopplungsschiene 17 (links bezogen auf Fig. 2) durchgeschoben sind. Das Messmodul 21 sowie das Therapiemodul 26 sind im noch nicht angedockten Zustand gezeigt.

Alle Messmodule 18 bis 21 weisen von den Modulköpfen 22 bis 25 ausgehend Messleitungen 27 bis 29 auf, an deren freien Enden wiederum Messsensoren 30 bis 32 beispielsweise für Druck, pH-Wert oder dergleichen angeordnet sind. Im Messmodul 18 ist der Temperatursensor 33 direkt in den Modulkopf 22 eingesetzt.

Das Therapiemodul 26 weist an seinem Modulkopf 34 einen Katheter 35 auf, der vor und an seinem freien Ende Stimulationselektroden 36, 37 trägt. Ferner ist ein Messsensor 38 beispielsweise für Druck oder Temperatur am Katheter 35 vorgesehen.

Zur Komplettierung des in Fig. 2 gezeigten Geräteimplantats ist noch eine Abschlusskappe 39 vorgesehen, die auf den letzten Modulkopf 34 aufgesetzt wird und zum Abschluss des durch die Modulköpfe 22 bis 25, 34 durchgeführten Bussystems 13 dient. Das Bussystem 13 wird über das Basisgeräteteil 15 und die Module 18 bis 21, 26 durch jeweilige Steckverbindungen 40 zwischen den Modulköpfen 22 bis 25, 34 und zum Basisgeräteteil 15 hin aufgebaut.

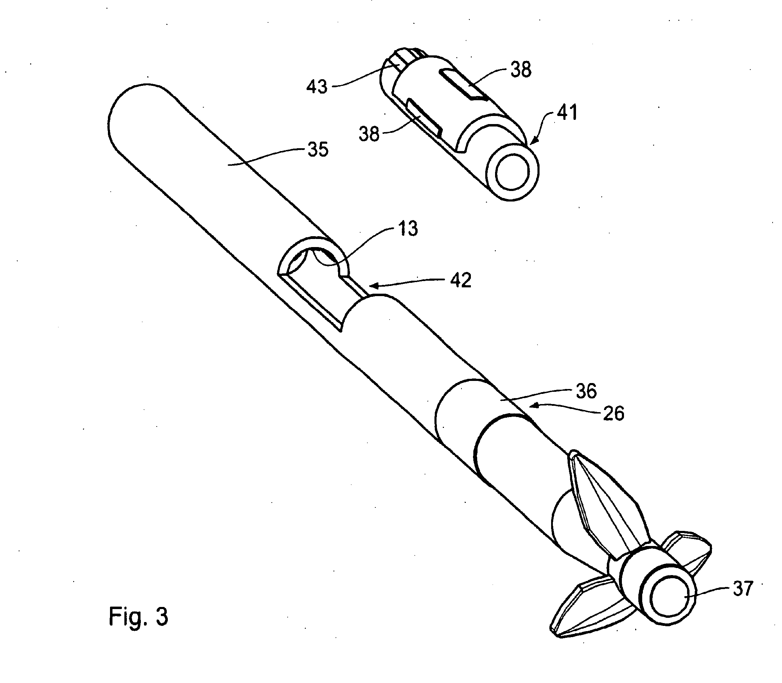

Fig. 3 zeigt ausschnittsweise den Katheter 35 im Bereich des distalen Endes. Daraus wird erkennbar, dass der Messsensor 38 wiederum selbst als austauschbares Teilmodul 41 ausgeführt sein kann. Dieses trägt - wie erwähnt - einen Messsensor für z. B. Druck oder Temperatur. Die Funktionalität des Teilmoduls 41 ist damit diagnostischer Natur, während die Stimulationselektroden 36, 37 am Therapiemodul 26 einen therapeutischen Effekt bewirken.

Das Teilmodul 41 ist in eine entsprechende Aufnahme 42 im Katheter 35 einsetzbar. Die Ankopplung an das (hier nicht dargestellte) Steuermodul erfolgt dabei über eine Steckverbindung 43 auf das Bussystem 13.

Zu ergänzen ist, dass die Module 18 bis 21, 26, 41 untereinander und mit dem zentralen Steuermodul über das Bussystem 13 uni-, bi- oder multidirektional kommunizieren können. Bei der Steuerung kann eine Master-and-Slave-Steuerung bezüglich der hierarchischen Ordnung der Module 18 bis 21, 26 implementiert werden.

## Patentansprüche

1. Aktives medizinisches Geräteimplantat mit mindestens zwei diagnostischen und/oder therapeutischen Funktionen, welches
- ein Basisgeräteteil (15) mit einer zentrale Steuereinheit (SE) und einem Bussystem (13); und
- für diese Funktionen jeweils gesonderte, miteinander über das Bussystem koppelbare, aktive Implantatmodule (18-21; 26) vorsieht,
wobei die Implantatmodule über das Bussystem mit der zentralen Steuerung uni-, bi- oder multidirektional kommunizieren und durch diese gesteuert werden, und wobei die Implantatmodule untereinander über das Bussystem uni-, bi- oder multidirektional kommunizieren können.

2. Geräteimplantat nach Anspruch 1, **dadurch gekennzeichnet, dass** die zentrale Steuereinheit (SE) bei der Steuerung eine Master-and-Slave-Steuerung bezüglich der hierarchischen Ordnung der Implantatmodule (18-21; 26) implementiert.

3. Geräteimplantat nach Anspruch 1, **dadurch gekennzeichnet, dass** das Basisgeräteteil (15) weitere funktionsübergreifende Grundelemente (KE, EE) für die Geräteimplantate (18-21, 26) aufnimmt.

4. Geräteimplantat nach Anspruch 3, **dadurch gekennzeichnet, dass** die weiteren funktionsübergreifenden Grundelemente eine Kommunikationseinheit (KE) und/oder eine Energieversorgungseinheit (EE) für die Implantatmodule (18-21, 26) als Grundelemente umfassen.

5. Geräteimplantat nach Anspruch 1, **dadurch gekennzeichnet, dass** die Implantatmodule (18-21, 26) zu ihrer Kopplung signaltechnisch und mechanisch mit dem Bussystem (13) verbindbar, vorzugsweise auf einer Kopplungsschiene (17) aneinanderreihbar sind.

6. Geräteimplantat nach Anspruch 5, **dadurch gekennzeichnet, dass** das Bussystem (13) durch die Implantatmodule (18-21, 26) durchgeführt und durch Aneinanderreihen von Implantatmodulen (18-21, 26) vorzugsweise über Steckkupplungen (40) erweiterbar ist.

7. Geräteimplantat nach Anspruch 1, **dadurch gekennzeichnet, dass** das Bussystem (13) zur signal- und kommunikationstechnischen Kopplung der Implantatmodule (18-21, 26) drahtlos ausgeführt ist.

8. Geräteimplantat nach Anspruch 7, **dadurch gekennzeichnet, dass** das drahtlose Bussystem auf Funkbasis, mittels induktiver Kopplung oder mittels galvanischer Kopplung über eine leitfähige Körperflüssigkeit realisiert ist.

9. Geräteimplantat nach einem der vorgenannten Ansprüche, **dadurch gekennzeichnet, dass** ein Implantatmodul (26) mit mindestens einem austauschbaren Teilmodul (41) vorzugsweise mit vom Implantatmodul (26) abweichender Funktionalität bestückbar ist.

10. Geräteimplantat nach einem der vorgenannten Ansprüche, **dadurch gekennzeichnet, dass** eines der Implantatmodule als Master-Gerät die Steuerung weiterer gekoppelter Implantatmodule übernimmt.

## Claims

1. An active medical device implant having at least two diagnostic and/or therapeutic functions, providing
- a basic device part (15) having a central control unit (SE) and the bus system (13); and
- active implant modules (18-21; 26), a separate one for each of these functions, connectable to one another via the bus system,
wherein the implant modules communicate with the central control unidirectionally, bidirectionally or multidirectionally via the bus system and are controlled by it, and wherein the implant modules can communicate with one another unidirectionally, bidirectionally or multidirectionally via the bus system.

2. The device implant according to claim 1,
**characterized in that**
the central control unit (SE) implements a master and slave control with regard to the hierarchical order of the implant modules (18-21, 26) in the control.

3. The device implant according to claim 1,
**characterized in that**
the basic device part (15) comprises additional function-overlapping basic elements (KE, EE) for the device implants (18-21, 26).

4. The device implant according to claim 3,
**characterized in that**
the additional function-overlapping basic elements comprise a communication unit (KE) and/or a power supply unit (EE) for the implant modules (18-21, 26) as basic elements.

5. The device implant according to claim 1,
**characterized in that**
the implant modules (18-21, 26) can be connected mechanically and by the signal technology to the bus system (13), preferably being aligned in a row on a coupling rail (17).

6. The device implant according to claim 5,
**characterized in that**
the bus system (13) is implemented by the implant modules (18-21, 26) and can be expanded by aligning implant modules (18-21, 26) in a row, preferably via plug connections.

7. The device implant according to claim 1,
**characterized in that**
the bus system (13) is designed for wireless coupling by signal technology and communications technology of the implant modules (18-21, 26).

8. The device implant according to claim 7,
**characterized in that**
the wireless bus system is implemented wirelessly, by inductive coupling or by galvanic coupling via a conductive body fluid.

9. The device implant according to any one of the preceding claims,
**characterized in that**
an implant module (26) can be assembled with at least one exchangeable partial module (41), preferably with a functionality that deviates from that of the implant module (26).

10. The device implant according to any one of the preceding claims,
**characterized in that**
one of the implant modules as the master device takes over the control of other coupled implant modules.

## Revendications

1. Implant d'appareil médical actif comprenant au moins deux fonctions de diagnostic et/ou thérapeutiques, qui prévoit
- une partie de l'appareil de base (15) avec une unité de commande (SE) centrale et un système de bus (13) ; et
- pour chacune de ces fonctions des modules d'implant (18-21 ; 26) actifs, séparés, pouvant être couplés les uns avec les autres par le système de bus,
les modules d'implant pouvant communiquer par le système de bus avec l'unité de commande centrale de façon unidirectionnelle, bidirectionnelle ou multidirectionnelle et étant commandés par celle-ci et les modules d'implant pouvant communiquer entre eux par le système de bus de façon unidirectionnelle, bidirectionnelle ou multidirectionnelle.

2. Implant d'appareil selon la revendication 1, **caractérisé en ce que** l'unité de commande (SE) centrale met en place lors de la commande une commande maître et esclave par rapport à l'ordre hiérarchique des modules d'implant (18-21 ; 26).

3. Implant d'appareil selon la revendication 1, **caractérisé en ce que** la partie de l'appareil de base (15) réceptionne d'autres éléments de base (KE, EE) recouvrant la fonction pour les implants d'appareil (18-21, 26).

4. Implant d'appareil selon la revendication 3, **caractérisé en ce que** les autres éléments de base recouvrant la fonction comprennent une unité de communication (KE) et/ou une unité d'alimentation en énergie (EE) pour les modules d'implant (18-21, 26) comme éléments de base.

5. Implant d'appareil selon la revendication 1, **caractérisé en ce que** les modules d'implant (18-21, 26) peuvent être reliés au système de bus (13) pour leur accouplement au niveau de la signalisation et de la mécanique, de préférence peuvent être alignés entre eux sur un rail d'accouplement (17).

6. Implant d'appareil selon la revendication 5, **caractérisé en ce que** le système de bus (13) est réalisé par les modules d'implant (18-21, 26) et peut être étendu par l'alignement de modules d'implant (18-21, 26), de préférence au moyen d'accouplements enfichables (40).

7. Implant d'appareil selon la revendication 1, **caractérisé en ce que** le système de bus (13) est réalisé sans fil pour l'accouplement au niveau de la signalisation et de la communication des modules d'implant (18-21, 26).

8. Implant d'appareil selon la revendication 7, **caractérisé en ce que** le système de bus sans fil est réalisé sur une base radio, par accouplement inductif et au moyen d'accouplement galvanique au moyen d'un liquide corporel conductible.

9. Implant d'appareil selon l'une quelconque des revendications précédentes, **caractérisé en ce qu'**un module d'implant (26) peut être équipé avec au moins un module partiel (41) échangeable de préférence avec une fonctionnalité différente du module d'implant (26).

10. Implant d'appareil selon l'une quelconque des revendications précédentes, **caractérisé en ce que** l'un des modules d'implant prend en charge en tant qu'appareil maître la commande d'autres modules d'implant couplés.
